# EUROPEAN PATENT APPLICATION

(11) **EP 4 809 969 A1**
(43) Date of publication of application: **23.09.2026**
(21) Application number: 26165620.1
(22) Date of filing: 17.03.2026
(51) Int. Cl.: A61C 7/00, A61C 13/12, G06T 7/00, A61B 5/145, A61C 13/02, A61C 13/01, G06F 3/048, G06T 7/12

(54) **METHODS FOR INTERPROXIMAL REDUCTION USING 3D SCANNER**

(30) Priority: 17.03.2025 US 202519081633
(71) Applicant: Derzapf, Evgenij, 64653 Lorsch (DE); Koza, André, 68163 Mannheim (DE); Bielser, Daniel, 3612 Steffisburg (CH); Wey, Peter, 5436 Würenlos (CH); Löschhorn, Sandro, 8172 Niederglatt (CH); Muff, Samuel, 8713 Uerikon (CH); Tanaka, Simon, 1010 Lausanne (CH); Petzoldt, Martin, 10243 Berlin (DE); Kaufmann, Markus, 13089 Berlin (DE); Dhondt, Stefaan, 3290 Diest (BE)
(72) Inventor: Derzapf, Evgenij, 64653 Lorsch (DE); Koza, André, 68163 Mannheim (DE); Bielser, Daniel, 3612 Steffisburg (CH); Wey, Peter, 5436 Würenlos (CH); Löschhorn, Sandro, 8172 Niederglatt (CH); Muff, Samuel, 8713 Uerikon (CH); Tanaka, Simon, 1010 Lausanne (CH); Petzoldt, Martin, 10243 Berlin (DE); Kaufmann, Markus, 13089 Berlin (DE); Dhondt, Stefaan, 3290 Diest (BE)
(74) Representative: Aldridge, Henry Alexander

(57) **Abstract**

Systems and methods for performing inter-proximal reduction (IPR) comprise removing a portion of a first tooth and scanning the modified portion with an intra-oral scanning system to obtain a partial scan. The system compares the modified portion to the original tooth to determine removed matter and provides real-time guidance about additional removal needed. The procedure can conclude by finalizing modifications according to the IPR procedure specifications. The system includes an intra-oral scanner for tooth imaging, a display screen, and a controller with stored instructions. The controller receives treatment plans with IPR specifications, processes scan imaging of modified teeth, compares scans to specifications for conformance, and generates guidance data about needed additional tooth matter removal. Guidance can be displayed through heat maps, color coding, or indicators projected directly onto teeth or via heads-up displays to guide precise tooth matter removal while preventing over-reduction.

## Description

### TECHNICAL FIELD

The present disclosure is generally directed to, but not by way of limitation, systems, devices and methods for performing orthodontic procedures, such as those related to using orthodontic alignment trays. More specifically, but not by way of limitation, the present disclosure is directed to systems, devices and methods for interproximal reductions for orthodontic alignment tray procedures.

### BACKGROUND

The field of orthodontics relates to the treatment of misalignment and malocclusion of teeth. Conventional methods for correcting alignment of teeth involve the use of braces, brackets, wires and the like, that can be adjusted by a dental professional, such as an orthodontist, to progress through a pathway for applying appropriate forces to move teeth into better alignment. As the teeth are moved in such conventional procedures, they will often be blocked by other teeth. Because of the initial misalignment, it is often not simply a matter of moving or repositioning a single tooth in one direction to correct its position. Rather, the treatment plan most often must account for physical contact with adjacent teeth as all of the teeth are collectively moved. For example, sometimes it is advantageous to remove small amounts of tooth matter, e.g., enamel, to facilitate alignment in a procedure called stripping or interproximal reduction (IPR). Even though effective alignment of the teeth can be ultimately achieved, some patients are not satisfied with the appearance of conventional orthodontic braces and the maintenance required of them.

More recently, methods of correcting the position of misaligned teeth involve using orthodontic alignment trays, also referred to as dental trays, aligner trays or aligners, fabricated in a manner that a given tray will itself exert a force upon the misaligned teeth to cause movement. These include for example, the SureSmile^{®} tray system from Dentsply Sirona, Inc. of Charlotte, North Carolina and the Invisalign^{®} tray system from Align Technology, Inc. of Santa Clara, California. These dental trays can be provided in a series such that each successive tray moves the teeth more or differently than the previous tray, in an incremental fashion so as to effect the prescribed treatment plan. Each dental tray, therefore, will move certain teeth from a starting or "before" position to a selected ending or "after" position. The "after" position for a given dental tray can be based solely upon the nature of the immediately previous "before" position. Further, the shape and force exerted by each successive dental tray in the treatment process of the conventional system can be based only upon the nature of where the previous dental tray left off in the moving of the teeth. Often these dental trays are fabricated from a clear plastic material and are disposed of after progressing to the next tray. Such dental trays are popular with patients for being more aesthetically pleasing and involving less maintenance.

Methods of employing dental tray systems can, however, suffer from similar difficulties as conventional orthodontic braces. There may be some target goal in mind as to where the dental professional wants to ultimately move the teeth, but until the very end of the treatment procedure, this final position and the initial starting position do not affect the incremental or intermediate treatment operations. Because of this, it has often been necessary to apply stripping to teeth in interproximal reduction (IPR) procedures in order to allow the teeth to move in the approximate direction desired. The results of excessive stripping are often not aesthetically appealing or even healthy for the stripped teeth. In any event, even though dental tray systems can eventually lead to adequately aligned teeth, they do not always result in the teeth moving to the aligned position in the planned manner.

Examples of orthodontic systems are described in: Pat. No. US 8,123,519 B2 to Schultz, titled "Method and System for Personalized Orthodontic Treatment"; Pat. No. US 9,101,329 B2 to Ertl, titled "Method for Determining the Position of an Intraoral Measuring Device"; Pat. No. US 9,345,557 B2 to Anderson et al., titled "Orthodontic Aligner Fabrication by Overlay Method"; Pat. No. US 11,412,993 B2 to Stalder et al., titled "System and Method for Scanning Anatomical Structures and for Displaying a Scanning Result"; Pat. No. US 11,534,265 to Khardekar et al., titled "Interproximal reduction Planning"; WO 2006/014935 A2 to Schultz, titled "Two Phase Invisible Orthodontics"; and KR10-2204990 to Lee Kyung-ah, titled " Method for Inter Proximal Reduction in digital orthodontic guide and digital orthodontic guide apparatus for performing the method."

### OVERVIEW

The present inventors have recognized, among other things, that problems to be solved in the planning of orthodontic alignment tray systems, as well as conventional orthodontic braces, involve the incorporation of inter-proximal reductions during the treatment plan. More specifically, inter-proximal reductions are performed at some future time during the treatment plan to correct malocclusions. The resulting inter-proximal reduction can adversely affect the remainder of the treatment plan if not performed with sufficient adherence to the treatment plan.

Orthodontic alignment tray systems comprise trays that are placed over teeth to gradually reposition the teeth from a misaligned state to an aligned state. The teeth are initially scanned to generate a three-dimensional (3D) model of the misaligned teeth. The orthodontic alignment trays are derived from the 3D model using computer planning systems. The initial alignment tray can be configured to move the teeth from the position of the original 3D model. Subsequently, successive iterations of dental trays are planned that gradually move the teeth from the misaligned state to the aligned state based on iterative updates to the 3D model. Numerous iterations of dental trays can be used to achieve the final result. As mentioned, previous orthodontic alignment tray systems have been closed such that IPR procedures have been performed on an ad hoc basis to fit the teeth to the treatment plan. However, more recently, performance of IPRs have been incorporated into the planning process to be used at predetermined points within the treatment plan. For example, the shape of the dental trays can be planned using computer-based planning systems that coordinate the movement of adjacent teeth, such as by ensuring adjacent teeth do not conflict with each other. The computer planning process can incorporate the performance of IPRs at strategic points to minimize the amount of tooth matter to be removed.

Even with the incorporation of computer planning, IPRs are still typically performed by hand and rely on the skill, judgment and experience of the orthodontist to perform the IPR, whether used in conjunction with orthodontic tray aligners or conventional orthodontic braces. In particular, the IPR must be performed according to the surgical plan in order for the previously-planned and subsequently-used alignment trays to be effective as expected. Current methods, particularly in complex cases with overlapping or rotated teeth, make it challenging, if not impossible, to accurately reduce tooth width as planned using hand-held files. This is especially problematic when teeth are not well-aligned or are completely rotated out of alignment. Orthodontists often rely on manual measurements using calipers or gauges, which may not provide accurate information about the actual reduction achieved due to positioning of the tooth relative to adjacent teeth, if they can even be placed in proper position to obtain a measurement at all. For example, the existing malocclusion may prevent a caliper from being used due to an adjacent tooth blocking placement of the caliper or a tooth having a fully exposed side may prevent a gauge from being used. Thus, issues in the performance of the IPR are not readily apparent at the time of performing the IPR and do not typically manifest until later dental trays are being used further along in the treatment plan. For such reasons, IPRs can be delayed to later points in the treatment plan or avoided altogether, which can result in sub-optimal final tooth placement. As such, some dental trays used later in the treatment plan rely on IPRs to be accurately performed earlier in the treatment plan.

Thus, the present inventors have recognized that traditional IPR approaches lack effective feedback on the amount of tooth reduction performed for extremely occluded teeth. For example, in performing manual filing processes and subsequently measuring with handheld, analogue measuring systems, there is a risk of removing too much tooth material, which cannot be reversed and may lead to complications. The present subject matter can provide solutions to these and other problems, such as by providing systems and methods for providing feedback in the performance of IPR procedures.

The systems and methods of the present disclosure can involve the use of real-time feedback at the time of performing an IPR to determine if the proper amount of tooth matter has been removed in the correct locations, whether used in conjunction with orthodontic tray aligners or conventional orthodontic braces. In examples, the hand-held scanning systems can be used to obtain imaging of the tooth being reduced and adjacent teeth. The particular tooth to be reduced can be automatically recognized in the imaging based on the treatment plan for comparison to other imaging. Imaging of the tooth can be compared to the treatment plan to provide feedback to the orthodontist or general practitioner relating to how much additional tooth matter is to be removed to conform the tooth to the treatment plan. In examples, digital models can display heat maps, numerical depth values and the like to show how much tooth matter is to be removed. In examples, the digital models can display the current tooth shape after some tooth matter has been removed to the desired tooth shape. In examples, specific guidance to remove more tooth matter and/or where to stop removing tooth matter can be displayed. In examples, a heads-up display or augmented reality goggles can be used to show instructions on the tooth for removing tooth matter during the IPR procedure. In examples, an automatically controlled orthodontic instrument can be used to remove tooth matter according to the treatment plan. As such, the systems and methods of the present disclosure can allow an orthodontist to precisely remove tooth matter to ensure the proper amount of tooth matter in the correct locations is removed to maintain treatment according to the treatment plan. For example, the orthodontist can iteratively remove tooth matter in order to gradually conform the tooth to the planned IPR, thereby avoiding over-removal of tooth matter. Furthermore, the systems and methods of the present disclosure involving performing intra-operative scanning during performance of an IPR can allow for the performance of IPRs in situations where IPRs were previously avoided or unable to be performed with handheld measuring instruments.

An example of subject matter (e.g., a method of performing an inter-proximal reduction procedure) may comprise removing a portion of a first tooth to form a modified portion of the first tooth, scanning at least the modified portion of the first tooth with an intra-oral scanning system to obtain a partial scan, comparing the modified portion of the first tooth of the partial scan to the first tooth before modification to determine how much tooth matter has been removed, receiving guidance from the intra-oral scanning system relating to additional tooth matter to be removed and finalizing modification of the first tooth according to the inter-proximal reduction procedure.

In an example, which may be combined with any one or more examples described herein, the subject matter optionally comprises conducting an initial scan of the first tooth before removing the portion of the first tooth to form the modified portion of the first tooth and comparing the modified portion of the first tooth of the partial scan to the first tooth in the initial scan.

In an example, which may be combined with any one or more examples described herein, the subject matter optionally comprises receiving a treatment plan including a specification for performing the inter-proximal reduction procedure on the first tooth and removing the portion of the first tooth according to the specification to form the modified portion of the first tooth and finalizing modification of the first tooth according to the specification.

In an example, which may be combined with any one or more examples described herein, comparing the modified portion of the first tooth of the partial scan to the first tooth before modification optionally comprises comparing the modified portion of the first tooth of the partial scan to the specification to determine conformance of the modified portion to the treatment plan.

In an example, which may be combined with any one or more examples described herein, comparing the modified portion of the first tooth to the specification optionally comprises generating a three-dimensional representation of the modified portion of the first tooth and receiving guidance from the three-dimensional representation of the modified portion relating to conformance of the modified portion to the specification.

In an example, which may be combined with any one or more examples described herein, receiving guidance from the three-dimensional representation optionally comprises displaying a three-dimensional model of at least a portion of the first tooth and displaying a region to be removed from the three-dimensional model.

In an example, which may be combined with any one or more examples described herein, the subject matter optionally comprises displaying a scale on the portion to be removed corresponding to a depth of tooth matter to be removed and the scale optionally comprises a color-coded heat map or a numerically coded map.

In an example, which may be combined with any one or more examples described herein, the three-dimensional model is optionally generated on a heads-up display or a video display screen.

In an example, which may be combined with any one or more examples described herein, receiving guidance from the three-dimensional representation optionally comprises comparing the partial scan to a full arch scan previously obtained during a planning phase for the inter-proximal reduction procedure and the full arch scan has been modified to account for stages of dental trays planned to be applied to the first tooth.

In an example, which may be combined with any one or more examples described herein, receiving guidance from the three-dimensional representation optionally comprises comparing the partial scan to a pre-IPR scan obtained just before performing the inter-proximal reduction procedure after stages of dental trays have been applied to the first tooth.

In an example, which may be combined with any one or more examples described herein, receiving guidance relating to the conformance of the modified portion optionally comprises projecting indicia onto the modified portion of the first tooth with a projection device and the indicia indicate tooth matter to be removed.

In an example, which may be combined with any one or more examples described herein, the projection device optionally comprises a handheld intra-oral scanner of the intra-oral scanning system or a head-mounted projector.

In an example, which may be combined with any one or more examples described herein, receiving guidance relating to the conformance optionally comprises receiving a notification that an actual amount of tooth matter that has been removed is within a tolerance band of a recommended amount of tooth matter to be removed.

In an example, which may be combined with any one or more examples described herein, removing a portion of the first tooth optionally comprises filing away tooth matter with a handheld file or removing tooth matter with an automated tooth modification instrument that receives instructions from the intra-oral scanning system to activate a motorized reciprocating tool to remove tooth matter until a planned depth of tooth matter has been removed.

In an example, which may be combined with any one or more examples described herein, finalizing modification optionally comprises further modifying the first tooth to fit the first tooth into a modification envelope defined by the specification or visually inspecting the modified portion of the first tooth to ensure conformance to the specification of the treatment plan.

In an example, which may be combined with any one or more examples described herein, removing the portion of the first tooth optionally comprises removing a portion of a side of a rotated tooth that is exposed and scanning at least the modified portion comprises scanning the side of the rotated tooth to obtain a thickness of tooth matter removed from the side of the first tooth.

In an example, which may be combined with any one or more examples described herein, the side of the rotated tooth is optionally located within a lingual cavity of a mouth in which the first tooth is located.

In an example, which may be combined with any one or more examples described herein, removing the portion optionally comprises removing a portion of a corner of a partially rotated tooth that is engaged with an adjacent tooth and scanning at least the modified portion comprises scanning the corner of the partially rotated tooth to obtain a thickness of tooth matter removed from the corner of the first tooth.

In an example, which may be combined with any one or more examples described herein, the subject matter optionally comprises removing a portion of the adjacent tooth that engages the corner of the partially rotated tooth.

In an example, which may be combined with any one or more examples described herein, scanning at least the modified portion is optionally performed in-office at an orthodontic facility.

In an example, which may be combined with any one or more examples described herein, finalizing modification optionally comprises manufacturing dental trays to move the first tooth according to the treatment plan and revising the treatment plan to update dental tray stagings with imaging data obtained from the partial scan to be used after performing the inter-proximal reduction procedure to account for the first tooth as modified.

An example of subject matter (e.g., a system for performing an inter-proximal reduction procedure) may comprise an intra-oral scanning system configured to obtain imaging of at least a first tooth of a patient, a display screen configured to display imaging from the intra-oral scanning system and a controller comprising a non-transitory computer-readable storage medium having instructions stored therein for causing the system to receive a treatment plan including a specification for performing an interproximal reduction procedure on a first tooth, receive imaging from the intra-oral scanning system representing at least a portion of the first tooth after removal of tooth matter during the inter-proximal reduction procedure, compare the imaging to the specification to determine conformance of the first tooth to the treatment plan and generate guidance data based on compared imaging indicating an amount of additional tooth matter to be removed to conform the first tooth to the specification.

In an example, which may be combined with any one or more examples described herein, the intra-oral scanning system optionally comprises a handheld scanner configured to be inserted into a mouth of a patient.

In an example, which may be combined with any one or more examples described herein, the display screen optionally comprises a heads-up display configured to overlay guidance information onto a view of the first tooth.

In an example, which may be combined with any one or more examples described herein, the intra-oral scanning system optionally comprises a projector configured to project guidance indicators directly onto the first tooth based on the guidance data.

In an example, which may be combined with any one or more examples described herein, the subject matter optionally comprises an automated filing tool having a motorized reciprocating file and the instructions are optionally configured to activate the motorized reciprocating file to remove tooth matter until a planned depth of tooth matter has been removed according to the guidance data.

In an example, which may be combined with any one or more examples described herein, the instructions are optionally configured to generate a three-dimensional model of the portion of the first tooth on the display screen and the guidance data optionally comprises at least one of: heat maps, color coding, or projected indicators showing locations and amounts of tooth matter to be removed that are displayed on the three-dimensional model of the portion of the first tooth shown on the display screen.

An example of subject matter (e.g., a non-transitory computer-readable storage medium) may comprise instructions stored therein for causing an intra-oral scanning system connected to a display screen to receive a treatment plan including a specification for performing an interproximal reduction procedure on a first tooth, receive imaging from the intra-oral scanning system representing at least a portion of the first tooth after removal of tooth matter during the inter-proximal reduction procedure, compare the imaging to the specification to determine conformance of the first tooth to the treatment plan and generate guidance data based on compared imaging indicating an amount of additional tooth matter to be removed to conform the first tooth to the specification.

In an example, which may be combined with any one or more examples described herein, the intra-oral scanning system comprises a handheld scanner configured to be inserted into a mouth of a patient.

In an example, which may be combined with any one or more examples described herein, the display screen comprises a heads-up display configured to overlay guidance information onto a view of the first tooth.

In an example, which may be combined with any one or more examples described herein, the intra-oral scanning system comprises a projector configured to project guidance indicators directly onto the first tooth based on the guidance data.

In an example, which may be combined with any one or more examples described herein, the intra-oral scanning system further comprises an automated filing tool having a motorized reciprocating file, wherein the instructions are configured to activate the motorized reciprocating file to remove tooth matter until a planned depth of tooth matter has been removed according to the guidance data.

In an example, which may be combined with any one or more examples described herein, the instructions are configured to generate a three-dimensional model of the portion of the first tooth on the display screen and the guidance data comprises at least one of: heat maps, color coding, or projected indicators showing locations and amounts of tooth matter to be removed that are displayed on the three-dimensional model of the portion of the first tooth shown on the display screen.

An example of subject matter (e.g., a computer-implemented method) may comprise receiving a treatment plan including a specification for performing an interproximal reduction procedure on a first tooth; receiving imaging from an intra-oral scanning system, the imaging representing at least a portion of the first tooth taken after removal of tooth matter during the inter-proximal reduction procedure; comparing the imaging to the specification to determine conformance of the first tooth to the treatment plan; and generating guidance data based on the compared imaging, wherein the guidance data indicates an amount of additional tooth matter to be removed to conform the first tooth to the specification.

In an example, which may be combined with any one or more examples described herein, the method further comprises displaying imaging from the intra-oral scanning system on a display screen, optionally wherein the display screen comprises a heads-up display configured to overlay guidance information onto a view of the first tooth.

In an example, which may be combined with any one or more examples described herein, the method further comprises generating a three-dimensional model of the portion of the first tooth on the display screen and the guidance data comprises at least one of: heat maps, color coding, or projected indicators showing locations and amounts of tooth matter to be removed that are displayed on the three-dimensional model of the portion of the first tooth shown on the display screen.

In an example, a system, method, or apparatus may optionally combine any portion or combination of any portion of any one or more of the examples described herein.

### BRIEF DESCRIPTION OF THE DRAWINGS

FIG. 1 shows a block diagram illustrating examples of fabricating and applying orthodontic alignment trays for orthodontic alignment procedures.
FIG. 2 is a side view of a skull showing a maxillary arch and a mandibular arch of teeth of a patient.
FIG. 3A is a perspective view of a digital model of the maxillary arch and the mandibular arch of the teeth of FIG. 2.
FIG. 3B is a top plan view of a digital model of the mandibular arch of FIG. 3A.
FIG. 4 is a top plan view of an aligner tray prepared from the digital model of FIG. 3B.
FIG. 5A is a plan view of an example arch of teeth wherein future proximal contact surfaces of a tooth to be reduced are already in contact with adjacent teeth, which presents a desirable situation for performing an interproximal reduction (IPR) procedure using currently available approaches.
FIG. 5B is a plan view of an example arch of teeth wherein future proximal contact surfaces of a tooth to be reduced are exposed, which presents an undesirable situation for performing an IPR procedure using currently available approaches.
FIG. 5C is a plan view of an example arch of teeth wherein one future proximal contact surface of a tooth to be reduced is exposed and another future proximal contact surface of the tooth to be reduced is in contact with an adjacent tooth, which presents an undesirable situation for performing an IPR procedure using currently available approaches.
FIG. 6 is a plan view of an example arch of teeth wherein both future proximal contact surfaces of a tooth to be reduced are exposed and one of such surfaces is overlapped with an adjacent tooth, which present an undesirable situation for performing an IPR procedure using currently available approaches.
FIG. 7 is a block diagram illustrating examples of performing a treatment plan using three-dimensional scanning to perform an interproximal reduction according to the present disclosure.
FIG. 8 is a schematic view of a scanning system suitable for use with the present disclosure.
FIG. 9A is a perspective view of a model of a tooth having an area to be removed by IPR highlighted in the model.
FIG. 9B is a perspective view of the model of the tooth of FIG. 9A showing the area to be removed having color coding to indicate how much tooth matter to remove.

### DETAILED DESCRIPTION

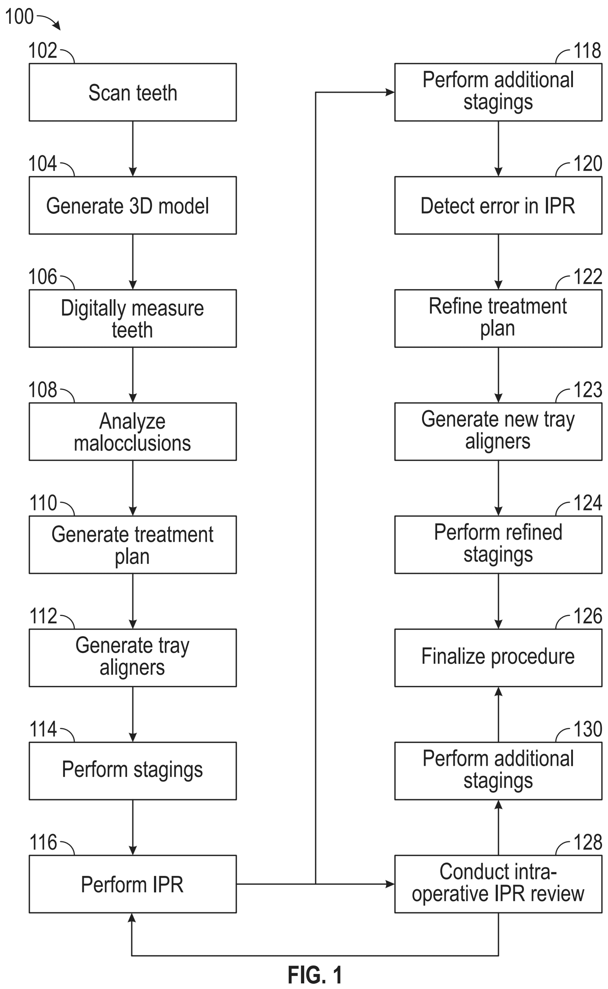

FIG. 1 shows block diagram illustrating operations of method 100 for fabricating and applying orthodontic alignment trays. FIG. 1 shows a conventional pathway for performing interproximal reductions in applying orthodontic alignment tray treatment plans as well as a pathway of the present disclosure. Although the present application is described with reference to use of orthodontic alignment trays, the systems, devices and methods described herein can be used with conventional orthodontic braces.

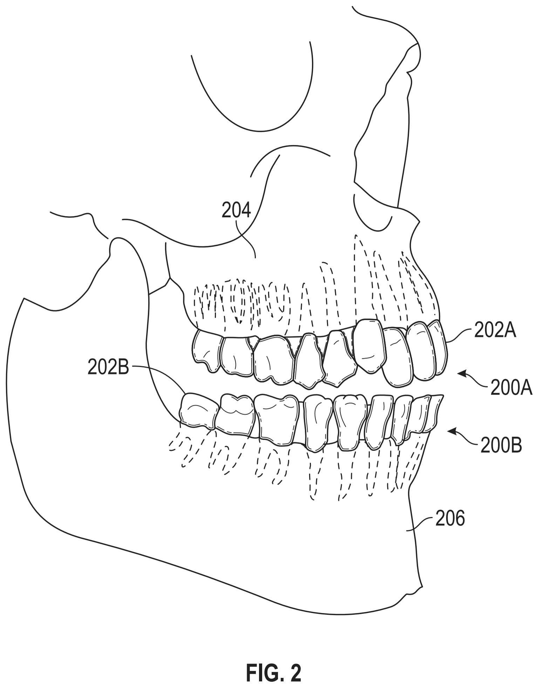

At operation 102, teeth 202A and teeth 202B (FIG. 2) of a patient can be scanned into a digital file, e.g., using a scanning software system. In examples, in-mouth scanning can be performed using small hand-held scanners. In examples, an intraoral scanning device as described in US 9,101,329 B2 to Ertl, titled "Method for Determining the Position of an Intraoral Measuring Device" can be used. In examples, impressions of teeth can be scanned using freestanding scanning systems, such as 3Shape R700, by 3shape A/S, of Copenhagen, Denmark (hereinafter referred to as 3shape). In additional examples, the OrthoPlex orthodontic digital modeling solution available from DENTSPLY International of York, Pennsylvania can be used. The OrthoPlex scanner and its associated software, can create a three-dimensional (3D) digital model that can be accessed by a computer or the like, and can be stored as an STL file. An STL file is a conventional format often used by stereolithography software to generate information needed to produce 3D models on stereolithography machines. While it is not necessary to use this file format, the format is useful to facilitate the use of fabricating orthodontic alignment trays using stereolithography or other rapid manufacturing processes.

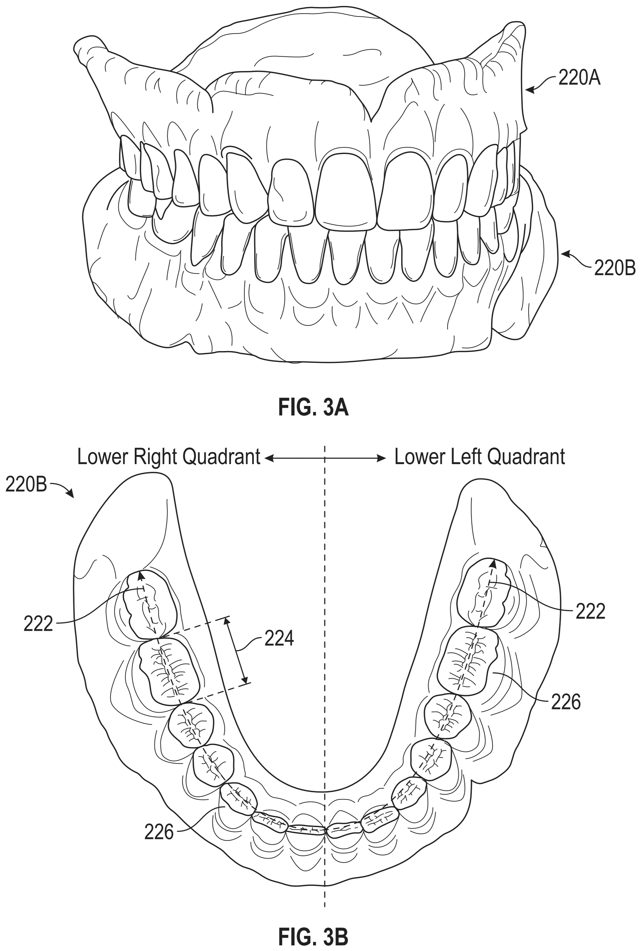

At operation 104, a 3D model of the teeth can be generated based on the scanned digital files. For example, maxillary arch model 220A and mandibular arch model 220B of FIG. 3A can be generated. The digital model can include the shape of each individual tooth of teeth 202A and teeth 202B as well as the placement of each tooth within maxilla 204 and mandible 206 (FIG. 2). As such, maxillary arch model 220A and mandibular arch model 220B can show the relative position and alignment, or misalignment, of each tooth of teeth 202A and each tooth of teeth 202B.

At operation 106, tooth width and arch width of the teeth can be measured digitally, e.g., using Ortho Analyzer software. Digital measurements can be taken of maxillary arch model 220A and mandibular arch model 220B. Computer-aided planning tools can be used to determine a complete three-dimensional coordinate set for all of teeth 202A and teeth 202B. For example, measurements of mesio-distal width 224 (FIG. 3B) of each tooth relative to mesio-distal direction 222 can be determined.

At operation 108, the positions of teeth 202A and teeth 202B (FIG. 2) can be analyzed to determine misalignment and malocclusion. For example, the amount of rotation of mesio-distal width 224 of each tooth relative to mesio-distal direction 222 can be determined.

At operation 110, a treatment plan can be generated. The treatment plan can be generated using software, e.g., Ortho Analyzer Next Generation software from 3shape or CEREC Ortho SW 2.0 software from Dentsply Sirona. The treatment plan can include the planning of the shape and size of a plurality of dental trays to be used to move the teeth from the starting position of the 3D model to a final, aligned position. The treatment plan can include the performance of one or more Inter Proximal Reductions (IPRs) at selected locations along the treatment plan, e.g., to be performed between particular dental tray iterations.

At operation 112, a plurality of dental trays or aligner trays can be fabricated to fit the treatment plan. The number of aligners can vary from case to case. In one exemplary embodiment, the aligner trays can be thermo-formed over a physical overlay model using, for example, an Essix vacuum forming machine and Essix Ace plastic thermoforming sheets. Both the sheets and the thermoforming machine are commercially available from Dentsply International, and the thermoforming process itself is conventional and known in the dental art. Using a physical overlay model to produce the aligner trays can result in a clear pathway of space from the teeth positions before patient treatment to the teeth positions after treatment, created to allow for tooth movement. As discussed, the pathway of space for the teeth positions can include the performance of one or more IPR procedures.

At operation 114, stagings with the aligner trays can be performed. For example, the aligner trays can be sent to an orthodontist. Thereafter the orthodontist can fit the aligner trays to the patient. The orthodontist can check each aligner tray in succession as the patient progresses through the teeth alignment process.

At operation 116, an orthodontist can perform a scheduled IPR. The IPR can be performed manually using hand-held tools, such as files, calipers, gauges and the like. Files can comprise a set of progressively thicker files. For example, really thin files can comprise sheets of flexible material configured to remove only a small amount of tooth matter, such as 0.7 millimeters (mm). In examples, files can be used having one or more of the following properties: 0.07 mm thickness, 0.10 mm thickness, 0.12 mm thickness, 0.15 mm thickness, 0.20 mm thickness, 0.25 mm thickness, 0.30 mm thickness, single-sided, double-sided, fine filing pattern, medium filing pattern, and coarse filing pattern.

From operation 116, method 100 can move through operation 118 to operation 124 to arrive at the final outcome at operation 126, or method 100 can move through operation 128 to operation 130 to arrive at the final outcome at operation 126. Operation 118 to operation 124 can represent a typical dental tray procedure wherein IPRs are performed and verified by manual processes, such as by using gauges, calipers and the like. Operation 128 and operation 130 can represent a dental tray procedure of the present disclosure wherein IPRs are performed and verified using intra-operative three-dimensional scanning of teeth.

At operation 118, additional stagings with the dental aligners can be performed after an IPR has been performed. At each staging, the orthodontist can check progress of tooth movement to the original treatment plan.

At operation 120, the orthodontist can detect an error or an abnormality in the treatment plan. For example, the patient can make in-office visits to the orthodontist so that the orthodontist can visually inspect teeth 202A and teeth 202B to see that one or more teeth may have not moved according to plan and/or that a dental tray will not fit properly with teeth 202A and teeth 202B. In examples, the orthodontist can determine that the IPR performed at operation 116 did not remove the appropriate amount of tooth matter and/or did not remove tooth matter in the appropriate location.

At operation 122, the treatment plan can be revised or refined to account for the current position of teeth 202A and teeth 202B to get the teeth back on track to move to the desired final position.

At operation 123, the refined treatment plan can include the generation of new dental aligners to move the currently situated teeth to a final position. The final position can be the previously determined final position or can be a new final position.

At operation 124, staging using updated dental trays produced to the refined treatment plan can be used.

At operation 126, the updated dental trays can be used according to the refined treatment plan to move teeth 202A and teeth 202B in the desired final alignment.

Operation 120 through operation 126 add extra time and cost to the performance of method 100. With the present disclosure, operation 120 through operation 126 can be avoided by the performance of operation 128. Furthermore, performance of operation 116 for the IPR can be moved to a more advantageous point in the treatment plan than between operation 114 and operation 118 so that the additional staging performed at operation 130 are more effective.

At operation 128, intra-operative imaging of teeth 202A and teeth 202B can be performed to obtain real-time images of teeth 202A and teeth 202B while an IPR procedure is being performed. The real-time images can be used to guide an orthodontist in removing tooth matter in the correct thicknesses and locations according to the treatment plan. As shown in FIG. 1, the real-time images can be used to remove tooth matter in an iterative fashion to avoid over-removal of tooth matter. Thus, an orthodontist can more accurately perform the IPR to maintain adherence to the treatment plan.

At operation 130, additional stagings can be performed. At each staging, the orthodontist can check progress of tooth movement to the original treatment plan. The dental trays for operation 130 can be those determined at operation 110 and operation 112 and do not need to be redesigned, such as at operation 122 and operation 124.

At operation 126, dental trays can be used according to the original treatment plan to move teeth 202A and teeth 202B to the desired alignment. As discussed, arrival at operation 126 via operation 128 and operation 130 can be advantageous over arrival at operation 126 via operation 118 through operation 124, such as by reducing time and cost to achieve final alignment, and potentially allowing for better alignment of teeth in the final position due to minimizing the amount of tooth matter removed, which can additionally result in a more aesthetically pleasing final alignment and appearance of the teeth.

FIG. 2 is a side view of a skull including maxillary tooth arch 200A and mandibular tooth arch 200B. Maxillary tooth arch 200A can comprise teeth 202A extending from maxilla 204. Mandibular tooth arch 200B can comprise teeth 202B extending from mandible 206. Maxillary tooth arch 200A can comprise a plurality of teeth 202A, such as molars, premolars, canines and incisors. Mandibular tooth arch 200B can comprise teeth 202B, such as molars, premolars, canines and incisors. In examples, some or all of teeth 202A can be situated in maxilla 204 in a misaligned manner, and/or some or all of teeth 202B can be situated in mandible 206 in a misaligned manner.

FIG. 3A is a perspective view of a digital model comprising maxillary arch model 220A and mandibular arch model 220B. Maxillary arch model 220A and mandibular arch model 220B can comprise nearly identical digital reproductions of maxillary tooth arch 200A and mandibular tooth arch 200B, respectively, including the alignment of teeth 202A and teeth 202B. However, multiple digital models can be produced to include gradual changing of the position of some teeth to correct the malocclusions, as discussed herein, to produce a series of dental alignment trays. The original 3D model that is an accurate representation of the current state of teeth 202A and teeth 202B can be modified by the orthodontic planning software at operation 110 to generate one or more modified 3D models corresponding to each staging of a dental tray. Thus, the digital models can form the basis for the production of tray aligner stagings.

FIG. 3B is a top plan view of mandibular arch model 220B of mandibular tooth arch 200B of FIG. 2. Each tooth of teeth 202B, as represented by model teeth 226, can have a mesial side that faces forward or the tooth in front of it and a distal side that faces backward or the tooth behind it. The buccal side of the tooth extends between the mesial side and the distal side and faces toward the cheek. The lingual side of the tooth extends between the mesial side and the distal side and faces toward the tongue. It is desirable from an aesthetics standpoint and from a functional standpoint to have each of teeth 202A and teeth 202B align such that the mesial sides and the distal sides are aligned. The term "mesio-distal width" of a tooth refers to the width or diameter of a tooth form as measured across the mesio-distal direction 222 of the dentulous arch form.

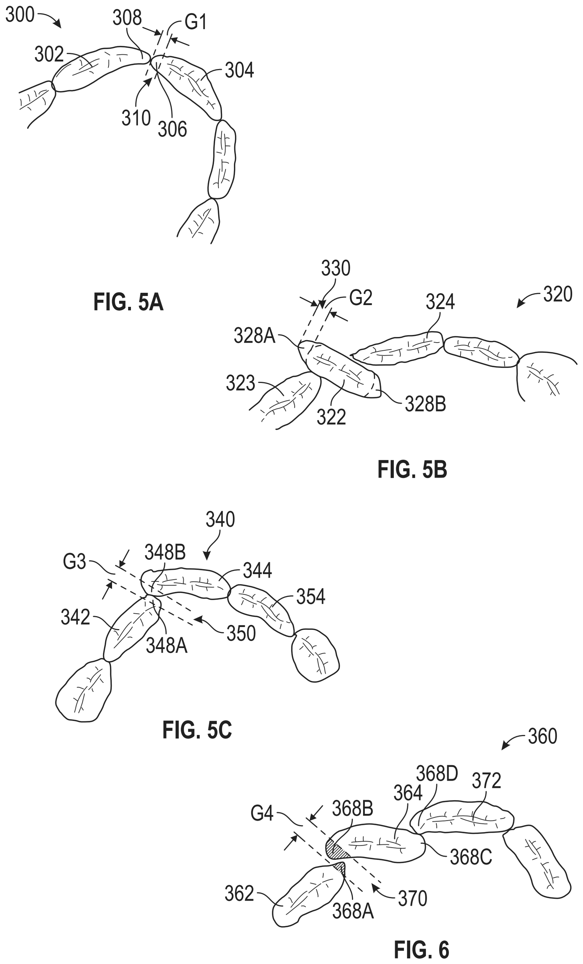

However, for a multitude of reasons, teeth 202B can grow into misaligned positions where in the mesial sides and the distal sides of the teeth are not suitably aligned along the mesio-distal direction 222. The malocclusions between teeth can have varying levels of misalignment that can dictate the treatment plan for the generation of aligner trays. In particular, the amount of rotation of one tooth relative to an adjacent tooth can determine if or when an interproximal reduction is in order. Different types of malocclusions are discussed with reference to FIG. 5A through FIG. 6.

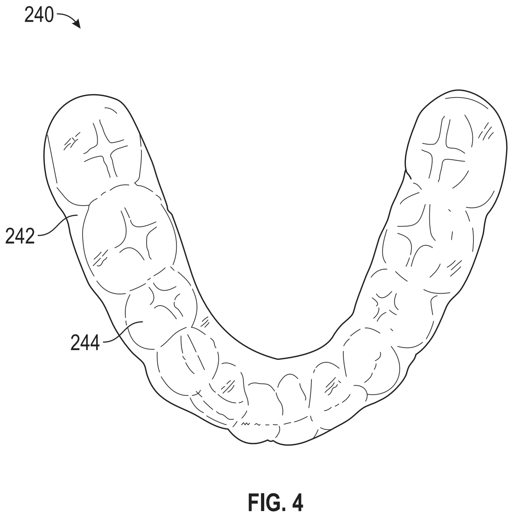

FIG. 4 is a top plan view of aligner tray 240 prepared from mandibular arch model 220B of FIG. 3B. Aligner tray 240 can include base 242 and pockets 244. Base 242 can comprise an arcuate trough shaped to fit around mandibular tooth arch 200B. Each pocket 244 can form a compartment for receiving one of teeth 202B. One, some or all of pockets 244 can be configured to apply pressure to a tooth of teeth 202B to gradually move the tooth into a different position according to the treatment plan. As discussed herein, a series of aligner trays similar to aligner tray 240 can be generated wherein the shape and/or size of one or more of pockets 244 changes from one staging to the next to gradually move the teeth to a desired position. The series of aligner trays can be designed around IPR procedures being performed between one or more stagings. In examples, aligner trays can be designed and manufactured according to those described in US. Pat. No. 8,123,519 to Schultz, titled "Method and System for Personalized Orthodontic Treatment," the entire contents of which are incorporated herein in their entirety by this reference.

FIG. 5A is a plan view of arch of teeth 300 wherein tooth 302 and tooth 304 are disposed for an interproximal reduction (IPR) procedure on tooth 304. In particular, tooth 304 can have region 306 that abuts against region 308 of tooth 302. The current rotational positions of tooth 302 and tooth 304, as illustrated in FIG. 5A, can be such that region 306 and region 308 will also be in contact at subsequent stages or phases of the treatment plan. However, the treatment plan can determine that the future position of the distal side of tooth 304 will undesirably contact the mesial side of tooth 302, after all of the alignments of teeth 300 are performed. Therefore, the treatment plan can call for an IPR to be performed on region 306 to prevent future contact between region 306 and region 308. As such, region 306, or a portion thereof, of tooth 304 can be reduced, e.g., removed, to facilitate future positioning of tooth 302 and tooth 304 with stagings of tray aligners. Because region 306 and region 308 are already in abutment, such a situation is well-suited for the performance of an interproximal reduction (IPR) procedure because a file can be extended along trajectory 310. For example, a one-sided file can be inserted in between tooth 302 and tooth 304 to remove region 306. A thin file can be used to make an initial gap between tooth 302 and tooth 304 and progressively thicker files can be used to achieve the desired gap G1 according to the treatment plan. Because the filing process results in a gap being formed between the mesial side of tooth 302 and the distal side of tooth 304, a gauge or a series of gauges can be readily used therebetween to measure how much tooth matter has been removed. Similarly, because the buccal and lingual sides of tooth 304 are exposed, the thickness of tooth 304 can be readily measured with a caliper. Thus, the situation of FIG. 5A lends itself to the performance of an IPR where region 308 presents a trajectory for a one-sided file to be used to remove region 308. Additionally, the situation of FIG. 5A is desirable for IPR if the treatment plan determines that a portion of region 306 and a portion of region 308 are to be removed to achieve the desired outcome. In such scenarios two-sided files can be used. As discussed herein, the IPR situation of FIG. 5A can additionally benefit from the use of intra-operative scanning to monitor the amount of tooth matter being removed from tooth 302 and tooth 304.

FIG. 5B is a plan view of an example arch of teeth 320 wherein tooth 322, tooth 323 and tooth 324 are disposed such that IPR is not readily achievable with conventional files, gauges and calipers. In particular, the current rotational position of tooth 322, as illustrated in FIG. 5B, is such that region 328A and region 328B are both exposed or uncovered and not contacting tooth 323 and tooth 324, respectively. However, it can be determined that region 328A will contact tooth 323 and region 328B will contact tooth 324 at a future point in the treatment plan when the mesio-distal orientation is corrected, taking into account all future movements of teeth 320 per the treatment plan. Thus, the treatment plan can determine that at least one of region 328A and region 328B of tooth 322 can be removed to facilitate future positioning of tooth 322, tooth 323 and tooth 304 with stagings of tray aligners. The situation of FIG. 5B does not lend itself to the performance of an IPR because trajectory 330 for region 328A is unsupported on one side and region 328B is difficult to reach with a file. Furthermore, even if one or both of region 328A and region 328B can be modified to achieve the desired size of tooth 322, it is not possible to measure tooth 322 with a gauge because the sides of tooth 322 are uncovered and there is nothing for a gauge to measure from. However, with the present disclosure, tooth 322 can be measured using intra-operative, three-dimensional scanning to verify the IPR procedure. Thus, the proper amount of region 328A and/or region 328B can be removed as discussed herein.

FIG. 5C is a plan view of arch of teeth 340 wherein tooth 342 and tooth 344 are disposed such that IPR is not readily achievable with conventional files, gauges and calipers. In particular, the current rotational position of tooth 344, as illustrated in FIG. 5C, is such that region 348B is exposed or uncovered and not contacting tooth 342 along the side. As can be seen in FIG. 5C, the lingual side of tooth 344 is contacting the mesial side of tooth 342. However, it can be determined that region 348A will contact region 348B of tooth 342 at a future point in the treatment plan when the mesio-distal orientation is corrected, taking into account all future movements of teeth 340 per the treatment plan. Thus, the treatment plan can determine that at least one of region 348A and region 348B of tooth 342 and tooth 344, respectively, can be removed to facilitate future positioning of tooth 342 and tooth 344 with stagings of tray aligners. The situation of FIG. 5C does not lend itself to the performance of an IPR because trajectory 350 for a file can remove portions of tooth 344 for which it is undesirable to remove. For example, trajectory 350 can remove too much of tooth 344 along the lingual side or can remove deeper tooth matter than the enamel layer. Additionally, trajectory 350 may not align with the way the teeth touch. Furthermore, even if one or both of region 348A and region 348B can be modified to achieve the desired size of tooth 342, it is not possible or very difficult to measure tooth 342 with a caliper because the side of tooth 344 abuts tooth 354. However, with the present disclosure, tooth 342 can be measured using intra-operative, three-dimensional scanning to verify the IPR procedure. Thus, the proper amount of region 348A and/or region 348B can be removed as discussed herein.

FIG. 6 is a plan view of arch of teeth 360 wherein tooth 362 and tooth 364 are disposed such that IPR is not readily achievable with conventional files, gauges and calipers. In particular, the current rotational position of tooth 364, as illustrated in FIG. 6, is such that region 368B is exposed or uncovered and not contacting tooth 362 along the side and region 368C is not contacting tooth 372 along the side. As can be seen in FIG. 6, the lingual side of tooth 364 is contacting the mesial side of tooth 362 and the buccal side of tooth 364 is contacting tooth 372. However, it can be determined that region 368A will contact region 368B and region 368C will contact region 368D of tooth 372 at a future point in the treatment plane when the mesio-distal orientation is corrected, taking into account all future movement of teeth 360. Thus, the treatment plan can determine that at least one of region 368A and region 368B of tooth 362 and tooth 364, respectively, can be removed to facilitate future positioning of tooth 362 and tooth 364 with stagings of tray aligners. The situation of FIG. 6 does not lend itself to the performance of an IPR because trajectory 370 for a file can remove portions of tooth 364 for which it is undesirable to remove. For example, trajectory 370 can remove too much of tooth 364 along the lingual side or can remove deeper tooth matter than the enamel layer. Additionally, trajectory 370 may not align with the way the teeth touch. Furthermore, even if one or both of region 368A and region 368B can be modified to achieve the desired size of tooth 362 or tooth 364, respectively, it is not possible or very difficult to measure tooth 362 with a gauge because the sides of tooth 364 are uncovered and there is nothing for a gauge to measure from. However, with the present disclosure, tooth 362 can be measured using intra-operative, three-dimensional scanning to verify the IPR procedure. Thus, the proper amount of region 368A and/or region 368B can be removed as discussed herein.

FIG. 5A through FIG. 6 illustrate common tooth situation encountered in performing orthodontic procedures. The situations of FIG. 5A through FIG. 6 present situations where IPR operations can be performed, whether orthodontic alignment trays or conventional orthodontic braces are used.

The present disclosure allows for the use of intra-operative, three-dimensional scanning to measure the subject teeth of an IPR procedure. Thus, even if a tooth has a side end that is uncovered where the use of a measurement gauge is not feasible because there isn't an abutting tooth for the gauge to measure from, the 3D scanning can accurately measure the subject tooth and how much tooth matter has been removed. Furthermore, even if a tooth is partially rotated such that one side abuts an adjacent tooth where a caliper cannot be used, the 3D scanning can still accurately measure the subject tooth and how much tooth matter has been removed.

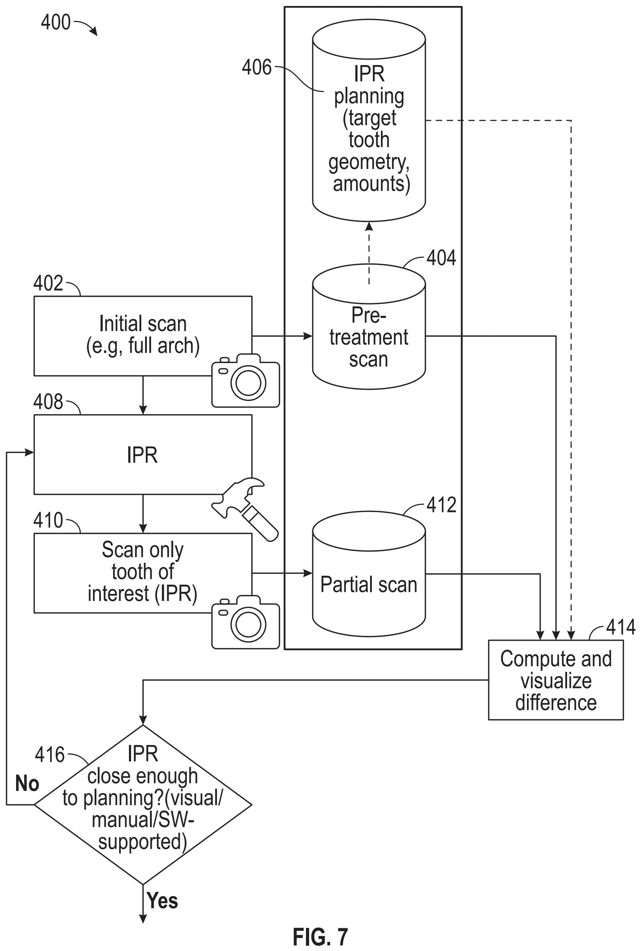

FIG. 7 comprises a block diagram of method 400 illustrating examples of performing a treatment plan using intra-operative, three-dimensional scanning to perform an interproximal reduction (IPR). Though discussed with reference to FIG. 1 through FIG. 6 and FIG. 8 through FIG. 9B, and a particular example of performing an intra-operative scanning process for an interproximal reduction, method 400 can encompass the use of any intra-operative scanning process and interproximal reduction procedure consistent with the methods and systems described herein. Method 400 can additionally include fewer or greater operations other than operation 402 to operation 416. Additionally, in other examples, operation 402 through operation 416 can be performed in other sequences.

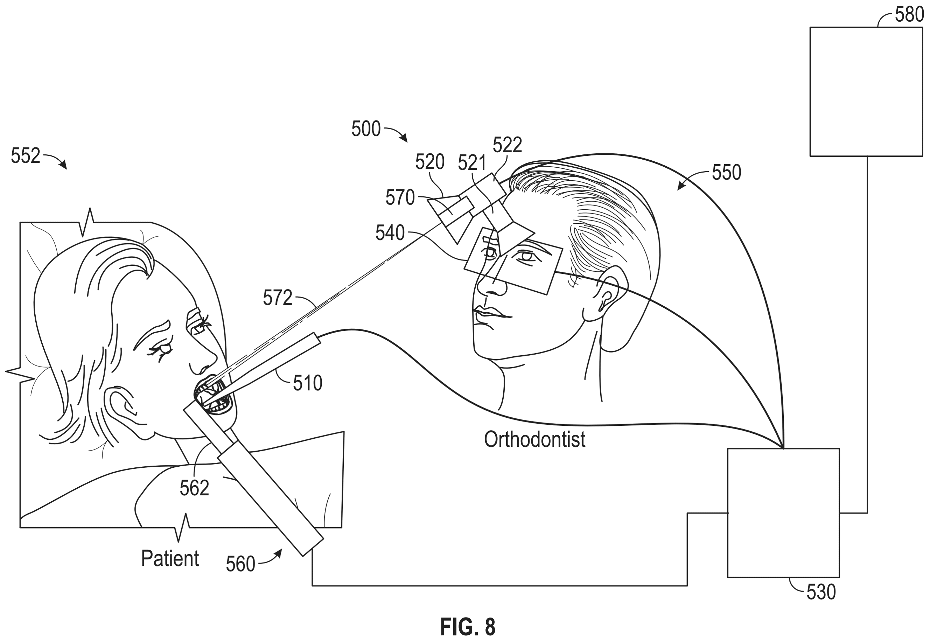

At operation 402, an initial scan of teeth of the patient can be captured by a scanner 612 to obtain a digital file including dimensions of the anatomic teeth. The mandibular and maxillary arches can be scanned. For example, maxillary tooth arch 200A and mandibular tooth arch 200B of FIG. 2 can be scanned to obtain images of teeth 202A and teeth 202B that can be converted into three-dimensional models. One or more three-dimensional, computer-generated models of the mandibular and maxillary arches can be generated and stored in computer readable storage medium, as can be connected to computing system 530 (FIG. 8). For example, maxillary arch model 220A and mandibular arch model 220B of FIG. 3A can be generated. The initial scan of the teeth can be performed using a hand-held, intra-oral scanning system as discussed herein.

At operation 404, the pretreatment scans, as well as three-dimensional models derived therefrom can be provided. The pretreatment scans and the three-dimensional may be provided to a provider for developing a treatment plan. The treatment plan can involve incremental movements of teeth from a current misaligned state to a final aligned state using a series of dental trays. For example, multiple instances of aligner tray 240 (FIG. 4), each being slightly modified from one another, can be used to adjust the position of teeth 202A and/or teeth 202B of maxilla 204 and mandible 206 (FIG. 2).

At operation 406, the pre-treatment scans can be used to generate a treatment plan, including the planning of tray aligners and IPRs. Furthermore, the treatment plan can be used to plan conventional orthodontic braces if desired. The treatment plans can involve the development of multiple dental trays as discussed herein to progressively and incrementally move and reorient the position of teeth. The treatment plan can additionally include the performance of IPRs at various stages within the treatment plan between applications of dental trays to facilitate the incremental movement of the teeth at acceptable intervals and to facilitate an aesthetically pleasing final position. Specific IPRs can be planned at operation 406 for specific teeth. The treatment plan can comprise a specification for performing an IPR procedure, such as original tooth shape, final tooth shape, locations for tooth material removal, amount of tooth material removal, tools to be used to perform the IPR and the like. The treatment plan can include specifications of thicknesses of tooth matter that is planned to be removed from particular teeth. For example, gap G1 on tooth 304 can be planned (FIG. 5A), gap G2 on tooth 322 can be planned (FIG. 5B), gap G3 on tooth 344 and tooth 342 can be planned (FIG. 5C), and gap G4 on tooth 364 and tooth 362 can be planned (FIG. 6). Specific values, e.g., thicknesses or depths of tooth matter to be removed, for gap G1, gap G2, gap G3 and gap G4 can be stored in the treatment plan and can be recorded digitally therein. Likewise, the location for gap G1, gap G2, gap G3 and gap G4, such as the side or sides of the tooth where the tooth matter is to be removed, can be stored in the treatment plan as part of the specification and can be recorded digitally therein.

At operation 408, an orthodontist or dental technician can perform an IPR on a patient. Before the IPR is performed, the orthodontist can scan the malocclusion of the patient to obtain reference images that can be compared to the treatment plan and the shape of the tooth that is to be achieved after the IPR is performed. In other examples, imaging from operation 402 and three-dimensional models of the tooth at such point in the treatment plan, e.g., a 3D model having the position of the teeth as moved by the dental trays at the point just before the IPR, can be used. The IPR can involve modification of a tooth by removing tooth matter or reducing the tooth by filing away layers of enamel of one or more teeth. In examples, manually operated files can be used. In examples, an automated filing device can be used, such as reduction instrument 560 of FIG. 8. The amount of tooth matter removed can be recorded in memory, e.g., memory or non-transitory storage media of computing system 530 (FIG. 8), for future reference. Operation 408 can coincide with operation 116 on FIG. 1. In examples, only part of the IPR can be performed. That is, a portion of the total amount of tooth matter to be removed can be removed before operation 410 is performed. As discussed below, method 400 can return to operation 408 after performing operation 410 to operation 416 to complete the IPR.

At operation 410, the orthodontist can perform a partial scan of the teeth of the patient, scanning only the tooth or teeth related to the IPR. For example, only the teeth being reduced can be scanned. However, in examples, adjacent teeth can be scanned, if desired. Operation 410 can coincide with operation 128 on FIG. 1. In examples, the orthodontist or another person can apply paint to the tooth upon which the IPR is to be performed to facilitate identification. The paint can be applied in areas of the tooth where tooth matter is not to be removed. The paint can be recognized by the scanning instrument to facilitate delineation of no-go areas. In examples, computing system 530 can provide a warning to a user if the wrong tooth is being scanned, e.g., if the scanned tooth does not match the tooth identified for the IPR in the treatment plan. In examples, only part of the IPR can be performed. In examples a hand-held scanning instrument can be inserted into a mouth of a patient to scan the tooth on which the IPR is being performed as well as adjacent teeth. The hand-held scanning instrument can be manipulated by an operator, e.g., an orthodontist, to obtain scans of multiple sides of the teeth. In examples, one or more of the scanning devices described with reference to FIG. 8 can be used, such as scanner 510. In examples, the scanning system can automatically identify the tooth or teeth being reduced, such as by comparing the scanned teeth to the treatment plan and images obtained at operation 402 and stored at operation 406.

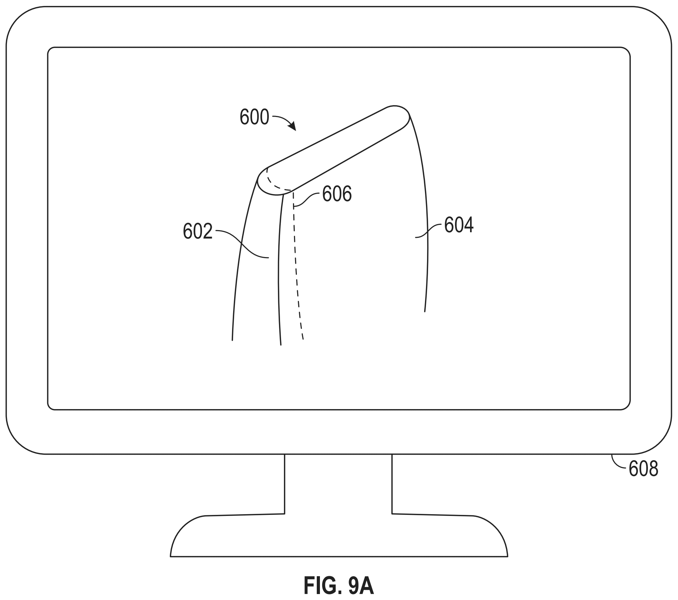

At operation 412, data files of the partial scan can be uploaded to a computing system having access to the original treatment plan. In examples, the data files can be provided to system 500 of FIG. 8. In particular, the partial scan can be displayed locally to the provider performing the IPR while the IPR is being performed. The partial scan, or a 3D model derived therefrom can be displayed on a display screen, such as a computer monitor, e.g., an LCD screen or LED screen, such as display screen 580 of FIG. 8 or display screen 608 of FIG. 9A. Additionally, the partial scan or the 3D model derived therefrom can be displayed on a heads-up display, such as screen 540 of FIG. 8. The computing system, e.g., computing system 530 of FIG. 8, can convert the data files for the partial scans, or raw data from a scanning device, into 3D models of the tooth or teeth.

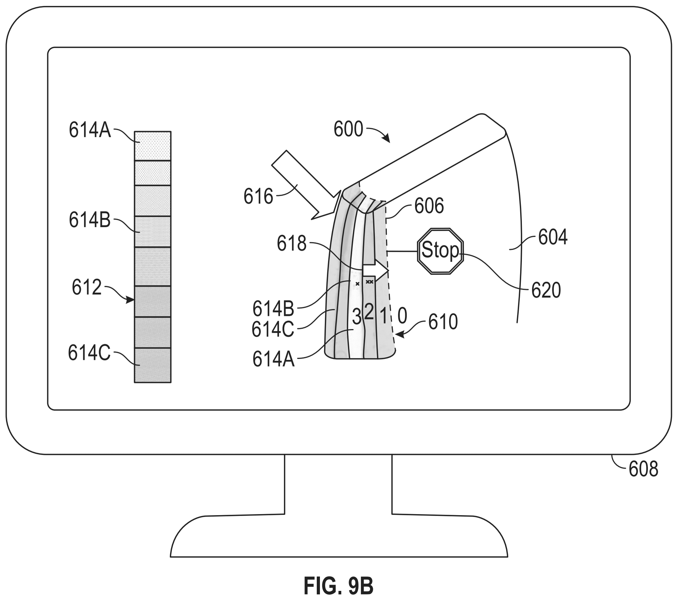

At operation 414, the performed IPR of operation 408 can be compared to the planned IPR of operation 406. Images of the teeth related to the IPR can be displayed with indicia or guidance related to how much tooth matter has been removed, how much additional tooth matter should be removed, how much excessive tooth matter has been removed and the like. In examples, computing system 530 of FIG. 8 can perform the comparisons locally. The 3D model developed from the 3D scan of the IPR or partial IPR can be compared to the digital model of the teeth for that particular stage of the dental tray staging or pretreatment scans taken at operation 404. The comparison can determine how much of the tooth matter planned to be removed at operation 406 has been removed so far. The comparison can involve subtracting tooth matter already removed to the total thickness of tooth matter to be removed, such as the values for gap G1, gap G2, gap G3 and gap G4 at operation 406. In examples, the comparison can involve comparing a target tooth shape to the planned tooth shape; in such case, every point or most points of the reduced tooth, not just the points where tooth matter is to be removed, can be compared to the planned shape. In either case, the comparison can involve displaying guidance related to whether or not the IPR being performed at operation 408 conforms with the IPR planned at operation 406. In examples, images similar to those discussed with reference to FIG. 9A and FIG. 9B can be displayed. In examples, guidance for how much tooth matter to be removed can be displayed on the teeth using projector 570 or scanner 510 (FIG. 8).

At operation 416, it can be determined if the performed IPR of operation 408 is sufficiently close to the planned IPR of operation 406. For example, it can be determined if the amount of tooth matter removed is within a tolerance band of tooth matter desired to be removed. For example, the digital model of the tooth to be reduced obtained at operation 410 can be checked by computing system 530 to see if it fits within the envelop of the tooth to be reduced planned at operation 406. If it is determined that additional tooth matter should be removed, method 400 can return to operation 408 to remove more tooth matter to more closely conform the tooth of the IPR to the planned, modified tooth. If it is determined that additional tooth matter is not to be removed, method 400 can conclude. In examples, method 400 can proceed to operation 130 of FIG. 1. The decision as to whether or not the IPR has been fully performed can either be made visually or manually by the orthodontist checking the visualization or supported by computing system 530 comparing to a target amount and/or target tooth shape. As such, computing system 530 can display appropriate audio, visual and tactile output to confirm the IPR has been completed. Additionally, computing system 530 can temporarily lock operation of reduction instrument 560 to prevent further removal of tooth matter until, for example, a user clears a warning presented on a display screen.

Furthermore, computing system 530 can update the treatment plan of operation 406 to update future stagings of dental trays to accommodate the shape of the teeth after the IPR is performed if, for example, the final tooth shape deviates from the treatment plan or an improved dental tray alignment path is detected. Additionally, computing system 530 can update the treatment plan to keep track of total cumulative tooth matter that has been removed for cases where a single tooth is planned to undergo multiple IPRs. Thus, removal of excessive tooth enamel can be prevented at future IPRs.

FIG. 8 is a schematic illustration of system 500 comprising scanner 510, detection unit 520, eye camera 521, position sensors 522, computing system 530, screen 540, reduction instrument 560 having removal tool 562, projector 570 having light beam 572 and display screen 580.

Screen 540 can comprise augmented reality glasses that can be placed directly into the field of view of a person conducting the scan, such as orthodontist 550. As such, the actual teeth of patient 552 and output of scanner 510 can be viewed at the same time on screen 540. Furthermore, as discussed with reference to FIG. 9A and FIG. 9B, cutting guidance can be displayed on teeth of patient 552 within screen 540. As such, the display unit comprising screen 540 can superimpose virtual content into the field of view of orthodontist 550 in an augmented reality format. In examples, screen 540 and eye camera 521 can be part of commercially available augmented reality systems, such as a Microsoft HoloLens, for example. In such a case, detection unit 520 for the spatial position of the scanner can be a 3D depth camera with an associated 2D color image. Additionally, computing system 530 can be integrated into the glasses.

Scanning results and reduction guidance, which are fitted into the actual, real-world surroundings, can be visible on screen 540. Output of scanner 510 (also referred to herein interchangeably as an intraoral scanner) are visible at a single glance and superimposed on one another. Screen 540 allows the user, the person conducting the scan, to see both the teeth as a scan template and the scanning results at the same time and superimposed on one another. Screen 540 can be designed in a variety of ways. In examples, screen 540 can be semi-transparent and virtual content can be superimposed on reality (a live view of the surroundings) and the live view of the surroundings appears at most slightly dimmed in an augmented reality configuration. In examples, screen 540 can be a completely virtual screen where virtual content is superimposed on a video of the surroundings in a virtual reality configuration. This video can be recorded from a natural point of view. The virtual content can, however, also be projected directly onto the retina of a person conducting the scan. Any combinations of the first example and the second example are also conceivable: the viewed surroundings can also be superimposed in a semi-transparent manner and/or superposition can be carried out by not showing the same content on the two screens in front of the eyes. The degree of superposition can be set individually by each user in examples.

The system further comprises detection unit 520, which can detect the spatial position of the intraoral scanner and is provided in direct proximity to screen 540, such as via a rigid connection to screen 540. A two-dimensional or a three-dimensional camera can be integrated into augmented reality glasses comprising part of system 500. This camera can function as detection unit 520 and can capture the scene from a point of view similar to that of the user of scanner 510 as the person conducting the scan. Detection unit 520 can be used to detect the spatial position of scanner 510 and to display the intraoral scanning result at a specific location, relative to the intraoral scanner. The virtual scanning result can be superimposed onto the real anatomical structures.

The system can further also comprise eye camera 521, which detects any movements of the eyes and/or the head of the person conducting the scan relative to detection unit 520 and screen 540. If, for example, the distance between the head and screen 540 is changed, a display can be adapted accordingly. If the viewing direction changes, the display can be changed as well.

The system can also include position sensors 522, which can detect movements of the user and help to display the contents of screen 540 in a stable manner.

System 500 can comprise scanner 510, which is configured as a two and/or three-dimensional, digital recording device. This can be executed in a variety of ways. The two-dimensional image can be obtained by means of an intraoral camera, for example. The three-dimensional model can be recorded with triangulation under structured illumination, with stereo cameras, confocally, by means of time-of- flight or other principles. In examples, scanner 510 can obtain a plurality of points that can be meshed together to provide a detailed shape of a tooth. In example, scanner 510 can be used to determine the texture of surfaces of the tooth to, for example, determine if enamel defines the outer layer or other tooth matter, such as dentin, cementum or pulp.

System 500 can comprise computing system 530, which connects scanner 510, screen 540 and detection unit 520, as well as eye camera 521 and position sensors 522, to one another. Computing system 530 can determine the superposition of scanning result and the live view, taking into account the viewing angle of the user. As discussed herein, computing system 530 can be provided with access to digital images, e.g., 3D models of teeth of a patient and various stagings in a treatment plan for orthodontic aligner trays and can be configured to compute differences from images obtained by scanner 510 to such 3D models to provide guidance for the removal of tooth matter to conform a tooth to the treatment plan. Computing system 530 can comprise a conventional computing device, such as a personal computer, a laptop, a tablet and a mobile phone, and can accordingly include appropriate processing units, circuitry, memory including random access memory and data storage devices, power supplies, wired and wireless communication devices, audio devices such as speakers, input and output devices such as a mouse and keyboard, and the like for executing instructions for the operation of scanner 510, reduction instrument 560, display screen 580 and the like according to the present disclosure.

Computing system 530 can be connected to a surgical instrument, such as reduction instrument 560. Computing system 530 can be configured to generate instructions for reduction instrument 560 based on comparison of the treatment plan to the current shape of the tooth to be reduced to, for example, control activation and deactivation of reduction instrument 560. In examples, reduction instrument 560 can comprise a reciprocating file wherein removal tool 562 comprises a filing surface configured to reciprocate relative to a handpiece via output of an electric motor. For example, scanner 510 can monitor operation of removal tool 562 and the amount of tooth matter being removed. If computing system 530 determines that the appropriate amount of tooth matter has been removed to complete the IPR, computing system 530 can disable or deactivate operation of reduction instrument 560 to reduce the possibility of excessive tooth removal occurring. However, the orthodontist can continue to manually remove tooth matter by manually reciprocation of removal tool 562. Additionally, an operator can clear or remove the block on operation of reduction instrument 560 by entering inputs into a user device, such as after acknowledging a warning indicating that computing system 530 indicates that adequate tooth matter has been removed to conform the IPR tooth to the treatment plan.

In additional examples, system 500 can comprise projector 570 configured to emit light beam 572. Light beam 572 can be configured to project indicia, e.g., colored light, directly onto teeth of patient 552. Additionally, scanner 510 can be configured to emit indicia, e.g., colored light, directly onto teeth of patient 552. Thus, light emitted by projector 570 or scanner 510 can be configured to instruct orthodontist 550 where to remove tooth matter based on scans obtained by scanner 510 that have been compared to the treatment plan. In examples, the indicia can comprise line 606 of FIG. 9A or the different colors of first zone 614A, second zone 614B and third zone 614C of FIG. 9B.

Computing system 530 can be connected to a display screen 580, which can comprise display screen 608 of FIG. 9A and FIG. 9B to display any output of scanner 510, digital models generated by output of scanner 510 and guidance for removing tooth matter, as discussed with reference to FIG. 9A and FIG. 9B. Any output displayed on screen 540 can additionally be displayed on display screen 580 and vice versa.

In examples, system 500 can be constructed according to Pat. No. US 11,412,993 B2 to Stalder et al., titled "System and Method for Scanning Anatomical Structures and for Displaying a Scanning Result," the entire contents of which are incorporated herein in their entirety by this reference. In examples, system 500 can be constructed according to Pat. No. US 9,101,329 to Ertl, titled "Method for Determining the Position of an Intraoral Measuring Device," the entire contents of which are incorporated herein in their entirety by this reference.

FIG. 9A is a perspective view of tooth 600 having area 602 to be removed by IPR. Tooth 600 can have surfaces 604 that form a three-dimensional shape. Area 602 can be delineated by line 606. Line 606 can show a portion of tooth 600 to be removed. In examples, area 602 can be located on the mesial side or the distal side of tooth 600. In the illustrated example, area 602 can comprise region 308 of FIG. 5A, but can additionally comprise region 328A of FIG. 5B, region 348B of FIG. 5C and region 368B of FIG. 6. Tooth 600 can be displayed on display screen 608.

In examples, tooth 600 can comprise the actual tooth of the patient being viewed by detection unit 520 of system 500 (FIG. 8). In such cases, line 606 can comprise visible light projected onto tooth 600 by system 500. In examples, tooth 600 can comprise a digital model generated by output of scanner 510 (FIG. 8). In such cases, line 606 can comprise coloration of pixels of the digital model generated by system 500. In either case, area 602 can comprise an area of tooth 600 to which an orthodontist is to apply a tooth matter removal tool, e.g., a file, to remove tooth matter from the actual tooth represented by tooth 600, e.g., tooth 304 (FIG. 5A), tooth 322 (FIG. 5B), tooth 344 (FIG. 5C) or tooth 364 (FIG. 6). Line 606 can delineate an envelope for which matter of tooth 600 is desired to be fit. Area 602 can be distinguished from the remainder of tooth 600 by being colored, patterned or textured to provide visual distinction.

FIG. 9B is a perspective view of tooth 600 of FIG. 9A showing area 602 to be removed having color coding 610 to indicate how much tooth matter to remove. Tooth 600 can be shown next to scale 612. Color coding 610 can include first zone 614A, second zone 614B and third zone 614C.

First zone 614A can comprise a light color indicating that a larger depth of tooth matter is to be removed. Third zone 614C can comprise a dark color indicating that a thin layer of tooth matter is to be removed. Second zone 614B can comprise an intermediate color indicating that an intermediate amount of tooth matter is to be removed. Additional zones can be included between first zone 614A and second zone 614B and between second zone 614B and third zone 614C. Additionally, the shading scale can be reversed such that light colors indicate a thin layer of tooth matter to be removed, etc. Scale 612 can include numbers, which can additionally be reproduced directly on tooth 600, that corresponds to a depth of tooth matter to be removed, providing the orthodontist with information for selecting a type of file to use. In examples, the numbers can be indicated in millimeters of tooth matter to be removed. FIG. 9B shows "3", "2", "1" and "0" that can correspond to 0.3 mm, 0.2 mm, 0.1 mm and 0.0 mm as an example. The color coding can provide the orthodontist with an indication of how much tooth matter is to be removed in various locations on tooth 600. The color coding provided in FIG. 9B can be applied to tooth 600 before the IPR is performed to help identify differences in tooth matter between the treatment plan and the current state to provide guidance to the orthodontist before any tooth matter is removed. The color coding of FIG. 9B can additionally be inverted to colorize regions which should not be reduced.

In examples, guidance information can be shown in display screen 608. For example, arrow 616 can show the trajectory for a file. Arrow 616 can correspond to trajectory 310 of FIG. 5A, trajectory 330 of FIG. 5B, trajectory 350 of FIG. 5C or trajectory 370 of FIG. 6. Additionally, arrows, such as arrow 618 can be shown directly on tooth 600 to show a filing direction. Tooth 600 can additionally include boundary information, such as stop sign 620, where filing should not be performed.

Display screen 608 can be configured to output indicia comprising scale 612 as well as the other indicia. Scale 612 and the other guidance indicia can reduce or eliminate the need for a person to evaluate or decode the three-dimensional modelling data. The bone matter depth values displayed on tooth 600 can be determined, for example, from a comparison of the current shape of tooth 600 to the desired final shape after performing an IPR determined by the treatment plan. In examples, colors displayed on tooth 600 can be updated in real-time or iteratively based on output of scanner 510. In examples, scanner 510 can be simultaneously used while tooth matter is being removed, such as with a file, to provide real-time feedback of the tooth removal process.

As discussed herein, system 500 can be used to provide intra-operative information, evaluation and feedback regarding tooth volume, including volumes of tooth matter to be removed at an IPR site. The feedback can comprise discrete information about the tooth volume size relative to other portions of a tooth that is not to be removed. The feedback can also comprise information, such as comparison to database information regarding whether or not a tooth removal site or volume is too close to inner tooth matter, such as by displaying different textures on tooth models. The feedback can be in the form of visual indicators, such as heat maps, risk scales, color coding, numbers and the like to inform an orthodontics team about how close to a treatment plan an IPR is progressing. Furthermore, the feedback can comprise discrete recommendations, such as no-go or go recommendations for removal of further tooth matter.

The present disclosure provides systems and methods for performing interproximal reduction (IPR) procedures using real-time 3D scanning feedback. Although the present application is described with reference to use of orthodontic alignment trays, the systems, devices and methods described herein can be used with conventional orthodontic braces. The present disclosure provides a plurality of benefits over prior art systems and methods.
1. Real-time Scanning System: The systems and methods of the present disclosure can utilize intraoperative 3D scanning to monitor tooth reduction during IPR procedures, and can provide immediate feedback to orthodontists on the amount and location of tooth matter being removed.
2. Digital Comparison & Guidance: The systems and methods of the present disclosure can compare real-time scans to treatment plans and desired tooth shapes, and display visual guidance through heat maps, color coding, or projected indicators to show where and how much tooth matter should be removed.
3. Iterative Process: The systems and methods of the present disclosure can allow orthodontists to gradually remove tooth matter while checking progress, which can help prevent over-reduction by providing continuous feedback.
4. Visualization Options: The systems and methods of the present disclosure can display guidance on heads-up displays, computer screens, or project directly onto teeth, which can therefore show comparisons between current tooth shape and the planned reduction.
5. Enhanced Precision: The systems and methods of the present disclosure can allow for accurate IPR procedures even in complex cases with rotated or overlapping teeth, which can provide verification that proper amounts of tooth matter have been removed in correct locations. Thus, the use of handheld gauges and calipers, which can present ambiguity in some situations, can be reduced or eliminated.

Overall, the systems and methods of the present disclosure can improve accuracy and predictability of IPR procedures, while reducing the risk of removing too much tooth matter, particularly in challenging cases where traditional measurement methods are difficult to use.

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventor also contemplates examples in which only those elements shown or described are provided. Moreover, the present inventor also contemplates examples using any combination or permutation of those elements shown or described (or one or more aspects thereof), either with respect to a particular example (or one or more aspects thereof), or with respect to other examples (or one or more aspects thereof) shown or described herein.

In the event of inconsistent usages between this document and any documents so incorporated by reference, the usage in this document controls.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The above description is intended to be illustrative, and not restrictive. For example, the above-described examples (or one or more aspects thereof) may be used in combination with each other. Other embodiments can be used, such as by one of ordinary skill in the art upon reviewing the above description. The Abstract is provided to comply with 37 C.F.R. § 1.72(b), to allow the reader to quickly ascertain the nature of the technical disclosure. It is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description as examples or embodiments, with each claim standing on its own as a separate embodiment, and it is contemplated that such embodiments can be combined with each other in various combinations or permutations. The scope of the invention should be determined with reference to the appended claims, along with the full scope of equivalents to which such claims are entitled.

## Claims

1. A system for performing an inter-proximal reduction procedure, the system comprising:
an intra-oral scanning system configured to obtain imaging of a first tooth of a patient;
a display screen configured to display imaging from the intra-oral scanning system; and
a controller comprising a non-transitory computer-readable storage medium having instructions stored therein for causing the system to perform the following steps:
receive a treatment plan including a specification for performing an interproximal reduction procedure on a first tooth;
receive imaging from the intra-oral scanning system, the imaging representing at least a portion of the first tooth after removal of tooth matter during the inter-proximal reduction procedure;
compare the imaging to the specification to determine conformance of the first tooth to the treatment plan; and
generate guidance data based on the compared imaging, wherein the guidance data indicates an amount of additional tooth matter to be removed to conform the first tooth to the specification.

2. The system of claim 1, wherein the intra-oral scanning system comprises a handheld scanner configured to be inserted into a mouth of a patient.

3. The system of claim 1 or 2, wherein the display screen comprises a heads-up display configured to overlay guidance information onto a view of the first tooth.

4. The system of any of claims 1-3, wherein the intra-oral scanning system comprises a projector configured to project guidance indicators directly onto the first tooth based on the guidance data.

5. The system of any of claims 1-4, further comprising an automated filing tool having a motorized reciprocating file, wherein the instructions are configured to activate the motorized reciprocating file to remove tooth matter until a planned depth of tooth matter has been removed according to the guidance data.

6. The system of any of claims 1-5, wherein the instructions are configured to generate a three-dimensional model of the portion of the first tooth on the display screen and the guidance data comprises at least one of: heat maps, color coding, or projected indicators showing locations and amounts of tooth matter to be removed that are displayed on the three-dimensional model of the portion of the first tooth shown on the display screen.

7. A non-transitory computer-readable storage medium having instructions stored therein for causing an intra-oral scanning system connected to a display screen to perform the following steps:
receive a treatment plan including a specification for performing an interproximal reduction procedure on a first tooth;
receive imaging from the intra-oral scanning system, the imaging representing at least a portion of the first tooth after removal of tooth matter during the inter-proximal reduction procedure;
compare the imaging to the specification to determine conformance of the first tooth to the treatment plan; and
generate guidance data based on the compared imaging, wherein the guidance data indicates an amount of additional tooth matter to be removed to conform the first tooth to the specification.

8. The non-transitory computer-readable storage medium of claim 7, wherein the intra-oral scanning system comprises a handheld scanner configured to be inserted into a mouth of a patient.

9. The non-transitory computer-readable storage medium of claim 7 or 8, wherein the display screen comprises a heads-up display configured to overlay guidance information onto a view of the first tooth.

10. The non-transitory computer-readable storage medium of any of claims 7-9, wherein the intra-oral scanning system comprises a projector configured to project guidance indicators directly onto the first tooth based on the guidance data.

11. The non-transitory computer-readable storage medium of any of claims 7-10, wherein the intra-oral scanning system further comprises an automated filing tool having a motorized reciprocating file, wherein the instructions are configured to activate the motorized reciprocating file to remove tooth matter until a planned depth of tooth matter has been removed according to the guidance data.

12. The non-transitory computer-readable storage medium of any of claims 7-11, wherein the instructions are configured to generate a three-dimensional model of the portion of the first tooth on the display screen and the guidance data comprises at least one of: heat maps, color coding, or projected indicators showing locations and amounts of tooth matter to be removed that are displayed on the three-dimensional model of the portion of the first tooth shown on the display screen.

13. A computer-implemented method, comprising:
receiving a treatment plan including a specification for performing an interproximal reduction procedure on a first tooth;
receiving imaging from an intra-oral scanning system, the imaging representing at least a portion of the first tooth taken after removal of tooth matter during the inter-proximal reduction procedure;
comparing the imaging to the specification to determine conformance of the first tooth to the treatment plan; and
generating guidance data based on the compared imaging, wherein the guidance data indicates an amount of additional tooth matter to be removed to conform the first tooth to the specification.

14. The computer-implemented method of claim 13, further comprising displaying imaging from the intra-oral scanning system on a display screen, optionally wherein the display screen comprises a heads-up display configured to overlay guidance information onto a view of the first tooth.

15. The computer-implemented method of claim 13, wherein further comprising generating a three-dimensional model of the portion of the first tooth on the display screen and the guidance data comprises at least one of: heat maps, color coding, or projected indicators showing locations and amounts of tooth matter to be removed that are displayed on the three-dimensional model of the portion of the first tooth shown on the display screen.
